# EUROPEAN PATENT APPLICATION

(11) **EP 2 325 227 A1**
(43) Date of publication of application: **25.05.2011**
(21) Application number: 09813171.7
(22) Date of filing: 09.09.2009
(51) Int. Cl.: C08G 63/16, C07D 307/58, C08G 18/42, C08L 101/16

(54) **POLYESTER, POLYURETHANE AND METHODS FOR PRODUCING SAME**

(30) Priority: 12.09.2008 JP 2008235504
(71) Applicant: Kabushiki Kaisha Toyota Jidoshokki, Kariya-shi, Aichi 448-8671 (JP); National University Corporation Tokyo University of Agriculture and Technology, Fuchu-shi Tokyo 183-8538 (JP); Nagaoka University of Technology, Niigata 940-2188 (JP); Forestry And Forest Products Research Institute, Tsukuba-shi, Ibaraki 305-8687 (JP)
(72) Inventor: TAKASHIMA, Hiroaki, Kariya-shi Aichi 448-8671 (JP); YAMAMOTO, Yusuke, Kariya-shi Aichi 448-8671 (JP); MASE, Kohei, Kariya-shi Aichi 448-8671 (JP); SHIMO, Toshihisa, Kariya-shi Aichi 448-8671 (JP); ABE, Tomokuni, Kariya-shi Aichi 448-8671 (JP); SHIGEHARA, Kiyotaka, Fuchu-shi Tokyo 183-8538 (JP); KATAYAMA, Yoshihiro, Fuchu-shi Tokyo 183-8538 (JP); MASAI, Eiji, Nagaoka-shi Niigata 940-2188 (JP); NAKAMURA, Masaya, Tsukuba-shi Ibaraki 305-8687 (JP); OTSUKA, Yuichiro, Tsukuba-shi Ibaraki 305-8687 (JP); OHARA, Seiji, Tsukuba-shi Ibaraki 305-8687 (JP)
(74) Representative: TBK
(86) International application number: PCT/JP2009/066050
(87) International publication number: WO 2010/030026

(57) **Abstract**

The invention provides biodegradable polyesters, polyurethanes, comprising 3-carboxymuconolactone as a structural unit, 3-carboxymuconolactone esters and a process for their production.

Polyesters comprising a repeating unit represented by the following formula (I): 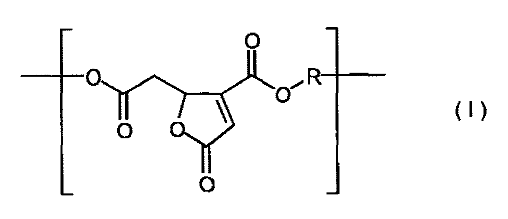 [wherein R represents a hydrocarbon-based divalent residue which, in the structure, optionally contains a heteroatom without an active hydrogen], polyurethanes comprising the polyester structure; 3-carboxymuconolactone esters; and a process for their production.

## Description

### Technical Field

The present invention relates to biodegradable polyesters, polyurethanes, comprising 3-carboxymuconolactone as a structural unit, 3-carboxymuconolactone esters and to a process for production thereof.

### Background Art

In recent years, increasing attention is being focused on vegetable-based resins that are made by plants such as corn or sugarcane. These kinds of resins cause less environmental disruption since they are biodegradable. Polylactic acid, polyhydroxybutyric acid and the like are known as such resins, and these have rigidity and strength equivalent to petroleum-based resins and are being developed for applications in various molding materials. However, because such vegetable resins use foodstuffs including starch and corn starch as raw materials, they are potentially in competition with food materials.

Lignins, on the other hand, are aromatic polymer compounds commonly found in plant cell walls, and they are vegetable resins that are not in competition with food materials. The vegetable components of lignins are converted to low molecular compounds such as vanillin, syringaldehyde, vanillic acid, syringic acid and protocatechuic acid by chemical decomposition methods such as hydrolysis and oxidative decomposition, or by physicochemical decomposition methods using supercritical water and supercritical organic solvents. Polymers obtained using such low molecular compounds as starting materials have been reported, such as a polyester and a polyamide comprising 2-pyrone-4,6-dicarboxylic acid (hereinafter abbreviated as PDC), which is a bacterial fermentation product of such compounds (International Patent Publication No. WO99/54376 and International Patent Publication No. WO99/54384, respectively). It is known that the 2-pyrone ring structure of PDC imparts a rigid structure to the polymer and can therefore provide flexibility, elasticity and high strength to the material, while its high polarity and high refractive index can yield materials having such properties (International Patent Publication No. WO99/54376, and International Patent Publication No. WO99/54384).

In addition, the present inventors have reported a process for industrial-scale fermentative production of 3-carboxymuconolactone, which can serve as a starting material for functional plastics and chemical products, by multistage enzyme reaction from a lignin-derived low molecular decomposition product (see Japanese Patent Application No. 2006-218524).

At the current time, however, the use of lignin-derived low molecular decomposition products other than PDC in polymers is unknown, and it has been desirable to develop a process for effective utilization of 3-carboxymuconolactone obtained from lignin-derived low molecular compounds.

### Disclosure of the Invention

The present invention therefore relates to biodegradable polyesters and polyurethanes comprising 3-carboxymuconolactone as a structural unit, and to a process for their production.

As a result of much diligent research in light of the situation described above, the present inventors have found that it is possible to easily and efficiently produce such a desired polyester by the polycondensation of 3-carboxymuconolactone or its salt or halide, with a diol or an alkali metal addition product thereof. It was also found that reacting the polyester with a diisocyanate can efficiently yield the desired polyurethane, and the invention has been completed upon this finding.

Specifically,
(1) the invention provides a polyester comprising a repeating unit represented by the following formula (I): 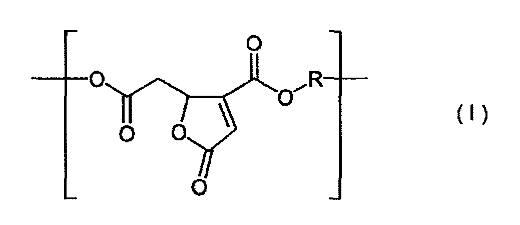 wherein R represents a hydrocarbon-based divalent residue which, in the structure, optionally contains a heteroatom without an active hydrogen.
(2) The invention provides a polyester according to (1), wherein R represents R¹, R¹- (OR¹)ₐ or R²-(O₂C-R¹-CO₂R²)_{b} (where R¹ and R² independently of each other represent a C1-24 saturated or unsaturated hydrocarbon divalent residue; and a and b independently of each other represent an integer of 1-4).
(3) The invention provides a polyester according to (1) or (2), wherein R is a C1-24 straight- or branched-chain alkylene group.
(4) The invention provides a polyurethane comprising a repeating unit represented by the following formula (II): 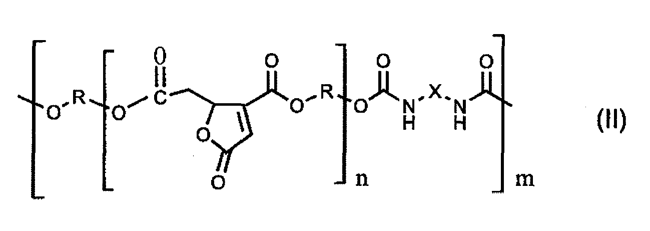 wherein
   R and X independently of each other represent a hydrocarbon-based divalent residue which, in the structure, optionally contains a heteroatom without an active hydrogen; and m and n each represent an integer of 1 or greater.
(5) The invention provides a polyurethane according to (4), wherein R and X independently of each other represent R¹, R¹-(OR¹)ₐ or R²-(O₂C-R¹-CO₂R²)_{b} (where R¹ and R² independently of each other represent a C1-24 saturated or unsaturated hydrocarbon divalent residue; and a and b independently of each other represent an integer of 1-4).
(6) The invention provides a polyurethane according to (4) or (5), wherein R and X are independently of each other a C1-24 straight- or branched-chain alkylene group.
(7) The invention provides a process for production of a polyester comprising a repeating unit represented by the following formula (I): 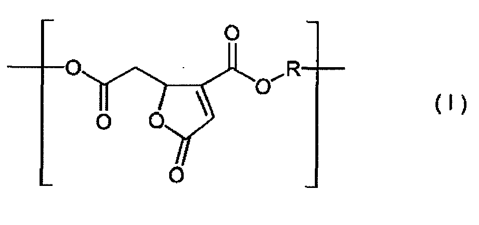 wherein R represents a hydrocarbon-based divalent which, in the structure, optionally contains a heteroatom without an active hydrogen,
   which comprises polycondensation of 3-carboxymuconolactone or its salt or halide, with a diol or an alkali metal addition product thereof.
(8) The invention provides a process for production of a polyurethane comprising a repeating unit represented by the following formula (II): 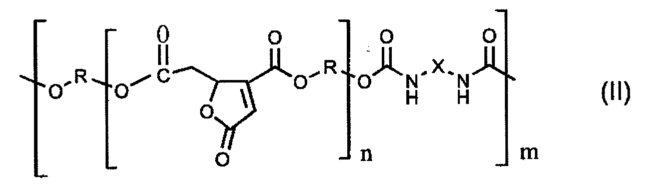 wherein R and X independently of each other represent a hydrocarbon-based divalent residue which, in the structure, optionally contains a heteroatom without an active hydrogen; and m and n each represent an integer of 1 or greater,
   in which a polyester obtained by the production process of (7) is reacted with a diisocyanate.
(9) The invention also provides a compound represented by the following formula (III): 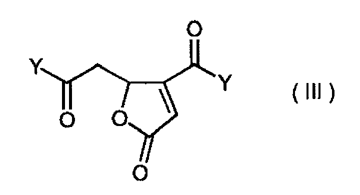 wherein Y represents a C1-6 alkoxy group, a halogen atom or a -O-R-OH group; and R represents a hydrocarbon-based divalent residue which, in the structure, optionally contains a heteroatom without an active hydrogen.

According to the invention it is possible to obtain biodegradable polyesters and polyurethanes.

### Best Mode for Carrying Out the Invention

The polyester of the invention has a repeating unit represented by formula (I), wherein R represents a hydrocarbon-based divalent residue which, in the structure, optionally contains a heteroatom without an active hydrogen, among which it preferably represents R¹, R¹- (OR¹)ₐ or R²-(O₂C-R¹-CO₂R²)_{b} (where R¹ and R² independently of each other represent a C1-24 saturated or unsaturated hydrocarbon divalent residue; and a and b independently of each other represent an integer of 1-4).

Examples of R¹ and R² include C1-24 straight- or branched-chain alkylene groups (such as ethylene, trimethylene, tetramethylene, hexamethylene, octamethylene, decamethylene and dodecamethylene), C3-8 cyclic alkane divalent residues (such as cyclohexylene), C6-20 aromatic hydrocarbon divalent residues (such as phenylene, tolylene, xylylene, naphthylene, methylnaphthylene and biphenylene), and C7-28 aralkyl divalent residues or C8-32 alkylarylalkyl divalent residues consisting of C1-6 alkyl groups and C6-20 aryl groups. As an example of the R¹-(OR¹)ₐ group there may be mentioned -CH₂CH₂-(OCH₂CH₂)₂-. As a R²-(O₂C-R¹-CO₂R²)_{b} group there may be mentioned -CH₂CH₂-(O₂C-CH₂CH₂-CO₂CH₂CH₂)-. These hydrocarbon-based divalent residues may optionally have additional substituents without active hydrogens, such as alkyl groups (preferably C₁-C₆ alkyl), alkoxy groups (preferably C₁-C₆ alkoxy), alkanoyl groups (preferably C₂-C₆ alkanoyl), aryl groups (preferably C₆-C₁₄ aryl) and aralkyl groups (preferably C₇-C₁₈ aralkyl).

C1-24 straight- or branched-chain alkylene groups are particularly preferred for R.

The polyester of the invention can be produced by polycondensation reaction between 3-carboxymuconolactone or a salt thereof (1) with a diol or its alkali metal addition product represented by the formula R⁴O-R-OR⁴ (2) wherein R is as defined in formula (I); and R⁴ represents an alkali metal element, according to the following reaction formula.

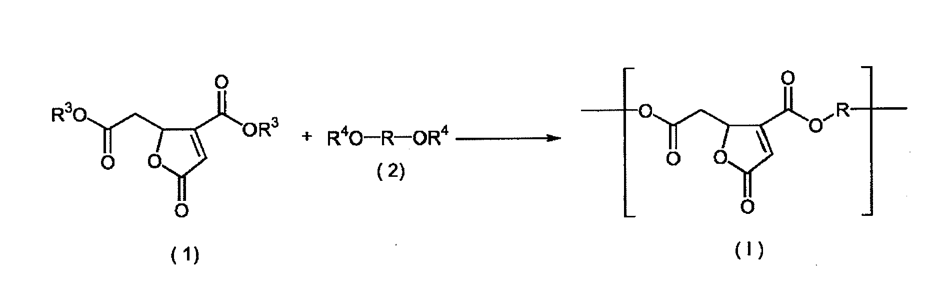

In the formula, R and R⁴ have the same definitions as above, and R³ represents hydrogen or an alkali metal element.

The compound 3-carboxymuconolactone can be conveniently produced by the method described in Japanese Patent Application No. 2006-218524, namely a method in which a transformant obtained by transferring a recombination vector comprising the vanillate demethylase gene, the benzaldehyde dehydrogenase gene and the protocatechuate 3,4-dioxygenase gene into Pseudomonas putida PpY1100 microorganisms is cultured in the presence of vanillin, vanillic acid, protocatechuic acid, or a mixture of two or more thereof.

Salts of 3-carboxymuconolactone include alkali metal salts, such as sodium salts, potassium salts and lithium salts.

Examples for the diol (2) include aliphatic diols such as ethylene glycol, propylene glycol, trimethylene glycol, 1,3-butanediol, 1,3-propanediol, 1,4-butanediol, 1,2-ethanediol, 1,5-pentanediol, 1,6-hexanediol, 1,7-heptanediol, 1,8-octanediol, 1,9-nonanediol, 1,10-decanediol, 1,4-cyclohexanedimethanol, trimethylolpropane, neopentyl glycol and methylpentanediol; as well as hydroquinone, catechol, 4,4'-dihydroxydiphenyl, 4,4'-hydroxybenzophenone, 4,4'-dihydroxydiphenylmethane, 4,4'-dihydroxydiphenylethane, 4,4'-dihydroxydiphenyl ether, 2,2-bis(4-hydroxyphenyl)propane, 4,4'-hydroxydiphenylsulfone, 4,4'-dihydroxydiphenyl sulfide, 2,6-dihydroxynaphthalene and 1,5-hydroxynaphthalene. Alkali metal addition salts of diols include sodium salts, potassium salts and lithium salts.

The mixing ratio of the 3-carboxymuconolactone or its salt (1) and the diol or its alkali metal addition salt (2) is preferably a molar ratio of approximately 1:1.

In the polycondensation reaction of the invention, it is possible to add an acid catalyst used for a common esterification reaction by dehydrating condensation. The acid catalyst may be, specifically, an inorganic acid such as hydrochloric acid, sulfuric acid or phosphoric acid, or an organic acid such as methanesulfonic acid, trifluoromethanesulfonic acid or p-toluenesulfonic acid. Instead of an acid catalyst, a condensation agent may be used, such as dicyclohexylcarbodiimide or diisopropylcarbodiimide. The amount of acid catalyst and/or condensation agent added will also differ depending on the concentration of the 3-carboxymuconolactone in the solution, but for most purposes it may be about 0.005-0.1 mol based on the 3-carboxymuconolactone. Dehydrating condensation may also be accomplished by conducting the reaction under reduced pressure (for example, 30 torr or greater), instead of using an acid catalyst or condensation agent. A combination of two or more acid catalysts and/or condensation agents may be used, and the acid catalyst and/or condensation agent may also be combined with reduced pressure conditions.

The polymerization solvent is not particularly restricted so long as it does not inhibit the polymerization reaction. Examples include ether-based solvents such as tetrahydrofuran, diethyl ether, 1,4-dioxane and dimethoxyethane; aromatic hydrocarbon-based solvents such as benzene, toluene and xylene; alicyclic hydrocarbon-based solvents such as cyclohexane and cyclohexanone; ester-based solvents such as acetic acid esters; and ketone-based solvents such as acetone and methyl ethyl ketone. These solvents may also be used in combinations of two or more. The amount of solvent used will normally be 20-1,000 parts by weight with respect to 100 parts by weight of the starting monomer.

The polymerization temperature will differ depending on the monomer used, the polymerization solvent and the reaction temperature, but for most purposes it is between room temperature and the boiling point of the solvent. The reaction time will also differ depending on the monomer used, the polymerization solvent and the reaction temperature, but for most purposes it will be from about several dozen hours to 200 hours.

There are no particular restrictions on the molecular weight of the polyester of the invention, but for most purposes it will be about 2,000-200,000 as the weight-average molecular weight, although this will differ depending on the purpose. From the viewpoint of ease of preparing the solution, molding workability and the physical properties such as mechanical strength, it is preferably about 10,000-100,000.

The urethane of the invention has a repeating unit represented by formula (II), wherein R is as defined in formula (I) and R³ has the same definition as R. In formula (II), R and R³ preferably independently of each other represent R¹, R¹-(OR¹)ₐ or R²-(O₂C-R¹-CO₂R²)_{b} (wherein R¹ and R² independently of each other represent a C1-24 saturated or unsaturated hydrocarbon divalent residue; and a and b independently of each other represent an integer of 1-4), and more preferably each represents a C1-24 straight- or branched-chain alkylene group.

There are no particular restrictions on the molecular weight of the polyurethane of the invention, but for most purposes it will be about 5,000-400,000 as the weight-average molecular weight, although this will differ depending on the purpose. From the viewpoint of ease of preparing the solution, molding workability and the physical properties such as mechanical strength, it is most preferably about 10,000-300,000.

The polyurethane of the invention may be produced by addition polymerization of a polyester represented by formula (I), and a diisocyanate (3) or its alkali metal addition product. A production example is illustrated below.

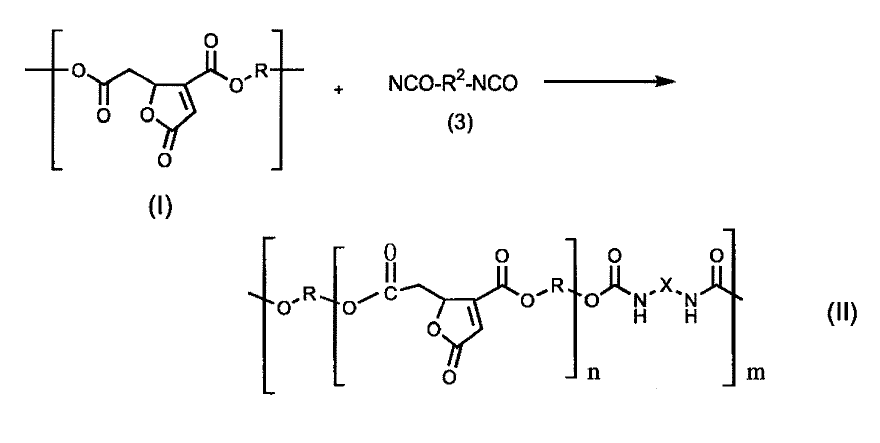

In the formula, R, R², X, m and n have the same definitions as above.

As examples of diisocyanates (3) there may be mentioned aromatic diisocyanates such as 4,4'-diphenylmethane diisocyanate, 2,4'-diphenylmethane diisocyanate, toluene diisocyanate (TDI), naphthalene diisocyanate and 1,4-phenylene diisocyanate; aliphatic diisocyanates such as ethylene diisocyanate, 2,2,4-trimethylhexamethylene diisocyanate and 1,6-hexamethylene diisocyanate (HDI); and alicyclic diisocyanates such as hydrogenated 4,4'-diphenylmethane diisocyanate (HMDI), 1,4-cyclohexane diisocyanate (CHDI), isophorone diisocyanate (IPDI), hydrogenated m-xylylene diisocyanate (HXDI), norbornane diisocyanate, xylylene diisocyanate (XDI) and tetramethylxylylene diisocyanate (TMXDI).

As alkali metal addition products of diisocyanates there may be mentioned potassium salts and sodium salts of the aforementioned diisocyanates.

The mixing ratio of the polyester (I) and diisocyanate (3) is not particularly restricted but is preferably about 2:1-1:3 as the molar ratio. If the diisocyanate is used in excess of this range, the isocyanates remaining on the polymer ends may react with the amine and cause off-odors or bad odors. In order to obtain a high molecular weight polyurethane, such as a polyurethane with a weight-average molecular weight of 100,000 or greater, the polyester (I) and diisocyanate (3) are preferably used in a molar ratio of about 1:1.

Although a polymerization catalyst is not absolutely required for production of a polyurethane of the invention, there may be employed catalysts ordinarily used for production of polyurethanes including, for example, tertiary amines such as triethylamine, tributylamine, N-methylmorpholine, N-ethylmorpholine, N,N,N',N'-tetramethylethylenediamine, pentamethyldiethylenetriamine, triethylenediamine, N-methyl-N'-dimethylaminoethylpiperazine, N,N-dimethylbenzylamine, N,N-dimethylcyclohexylamine, N,N,N',N'-tetramethyl-1,3-butanediamine and 1,2-dimethylimidazole; secondary amines such as dimethylamine; alkanolamines such as N-methyldiethanolamine, N-ethyldiethanolamine and N,N-dimethylethanolamine; tetraalkylammonium hydroxides such as tetramethylammonium hydroxide and benzyltrimethylammonium hydroxide; alkali metal phenolates such as sodium phenolate; alkali metal hydroxides such as potassium hydroxide; alkali metal alkoxides such as sodium methoxide; alkali metal salts of carboxylic acids such as potassium acetate, sodium acetate and potassium 2-ethylhexanoate; phosphines such as triethylphosphine; metal chelate compounds such as potassium-salicylaldehyde; organic tin (II) compounds such as stannous acetate and stannous octoate (stannous 2-ethylhexoate); organic tin (IV) compounds such as dibutyltin oxide, dibutyltin dichloride, dibutyltin diacetate, dibutyltin dilaurate, dibutyltin maleate and dioctyltin diacetate; and organometallic compounds such as dialkyl titanates. Isocyanuratated catalysts such as tris(dimethylaminomethyl)phenol and N,N',N'-tris(dimethylaminopropyl)hexahydro-s-triazine may also be used. These catalysts are preferably used at about 0.001-1 wt% in the reaction mixture.

As examples of solvents there may be mentioned ether-based solvents such as tetrahydrofuran, diethyl ether, 1,4-dioxane and dimethoxyethane; aromatic hydrocarbon-based solvents such as benzene, toluene and xylene; alicyclic hydrocarbon-based solvents such as cyclohexane and cyclohexanone; ester-based solvents such as acetic acid esters; ketone-based solvents such as acetone and methyl ethyl ketone; and aprotic polar solvents such as acetonitrile, N,N-dimethylformamide and dimethyl sulfoxide. These solvents may also be used in combinations of two or more. The amount of solvent used will normally be 20-1,000 parts by weight with respect to 100 parts by weight as the total starting monomer.

The polymerization reaction may be carried out for 1 hour to several hours at between 0°C and room temperature, optionally by heating the reactant.

The polyester and polyurethane of the invention may also be used in addition with various additives such as antioxidants, coloring agents, ultraviolet absorbers, light stabilizers, silane coupling agents, storage stabilizers, plasticizers, lubricants, solvents, fillers, age inhibitors, wettability improvers, coating surface improves and the like, as necessary.

The polyester and polyurethane of the invention are useful for various purposes including sheets, films, belts, hoses, vibration-proof materials, shoe soles, artificial leather, synthetic leather, fiber treatment agents, coating materials, adhesives, waterproof materials, elastic fibers, flooring materials and the like.

The compounds represented by the following formula (III) of the invention: 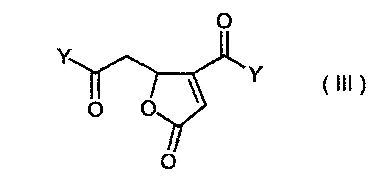 wherein Y represents a C1-6 alkoxy group, a halogen atom or a -O-R-OH group; and R represents a hydrocarbon-based divalent residue which, in the structure, optionally contains a heteroatom without an active hydrogen are novel compounds, and Y is preferably a methoxy, ethoxy or n-propoxy group, when it is a C1-6 alkoxy group, or fluorine, chlorine or bromine, when it is a halogen atom.

R may be a C1-6 alkylene group (such as ethylene, propylene, butylene or hexylene), a C6-12 arylene group (such as 1,4-phenylene), an aralkylene group consisting of a C1-6 alkylene group and C6-12 arylene group (such as benzylene) or a C6-18 cyclic fused group (such as 4,4'-biphenylene). The cyclic fused group may have a C1-6 alkylene group between the rings. These C1-6 alkylene and C6-12 arylene groups, aralkylene groups consisting of C1-6 alkylene and C6-12 arylene groups, and C6-18 cyclic fused groups, may be optionally substituted with C1-6 alkyl groups, C1-6 alkoxy groups or the like at the hydrogens of the alkylene groups and/or the hydrogens of the arylene groups.

The group -O-R-OH is preferably -O-CH₂CH₂-OH, -O-(CH₂CH₂)₂-OH or -O-(CH₂CH₂)₃-OH.

Of the compounds of formula (III), esters may be obtained, for example, by reacting 3-carboxymuconolactone with an alcohol (such as methanol, ethanol or n-propyl alcohol), a polyalkylene glycol (such as ethylene glycol or propylene glycol), a polyalkylene oxide (such as ethylene oxide or propylene oxide) and the like as necessary, in the presence of an acid catalyst such as hydrochloric acid, sulfuric acid or p-toluenesulfonic acid, while heating. A solvent that is inert to the reaction may also be added if necessary. Of the compounds of formula (III), acid halides can be obtained, for example, by reacting 3-carboxymuconolactone with a halide such as oxalic acid dichloride or thionyl chloride, in an appropriate solvent such as acetonitrile or tetrahydrofuran.

Of the esters represented by formula (III) it is preferred to use those formed from 3-carboxymuconolactone and polyalkylene glycols, for the production of a polyester of the invention.

### Examples

The present invention will now be further explained by production examples, with the understanding that the invention is not limited to the production examples. The physical properties of the obtained polymers were measured by the following methods.
(1) Thermogravimetry: The temperature at which the weight decreased from the initial weight upon temperature increase from 50°C at a temperature-elevating rate of 10°C/min under a nitrogen atmosphere was measured using a thermogravimetric analyzer (TGA) (TG/DTA220: product of SII Nanotechnology, Inc.).
(2) Weight-average molecular weight: Molecular weight measurement was performed by gel permeation chromatography GPC) (HLC-8220, product of Tosoh Corp.). Calibration was performed using standard polystyrene, and the weight-average molecular weight was determined based on polystyrene.
(3) Glass transition temperature: This was measured by differential scanning calorimetry (DSC) (DSC220, product of SII Nanotechnology, Inc.), with temperature increase at a temperature-elevating rate of 10°C/min.

### Example 1: Production of methyl 2-(2-methoxy-2-oxoethyl)-5-oxo-2,5-dihydrofuran-3-carboxylate

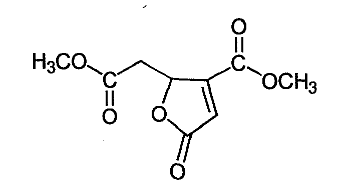

In a 25 ml flask there was placed 3-carboxymuconolactone (1.69 g, 9.00 mmol), and then 10 ml (an excess) of methanol was added and the mixture was stirred and dissolved. The 3-carboxymuconolactone was obtained by the method described in Japanese Patent Application No. 2006-218524. After completely dissolving the 3-carboxymuconolactone, several drops of concentrated sulfuric acid were added as an acid catalyst and the mixture was stirred for 9 hours under reflux at 100°C, under an air atmosphere. The obtained solution was dissolved in a sodium hydrogencarbonate aqueous solution, chloroform was added and the organic layer was extracted (x 2). The obtained organic layer was dried over magnesium sulfate, and the organic layer was distilled off under reduced pressure to obtain 1.43 g of the title compound (yield: 73.6%).

¹H-NMR (500 MHz, CDCl₃) δ (ppm) : 2.77 (1H q), 3.18(1H q), 3.73(3H s), 3.93(3H s), 5.56 (1H m), 6.73 (1H d).

¹³C-NMR (500 MHz, CDCl₃) δ (ppm): 37.00(lactone ring 5-position α), 52.24(methyl group), 53.00(methyl group), 78.34(lactone ring), 127.48(lactone ring), 155.78(lactone ring), 161.19(carboxyl group), 169.12(carboxyl group), 170.13(lactone ring).

GC-MS m/z: 214 (M⁺), 182, 154, 141, 113.

### Example 2: Production of polyester

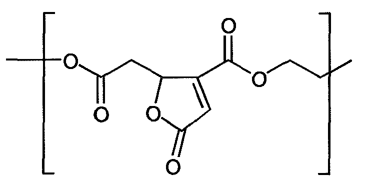

In a 25 mL flask there was placed 3-carboxymuconolactone (3.57 g, 19.2 mmol), and then 7 ml of tetrahydrofuran was added and the mixture was dissolved while stirring. After completely dissolving the 3-carboxymuconolactone, ethylene glycol (1.19 g, 19.2 mmol) was added thereto, the mixture was stirred for 5 minutes, several drops of concentrated hydrochloric acid were added as an acid catalyst, and the mixture was stirred for several days at 100°C. The obtained solution was dried at 60°C in a vacuum to obtain 3.72 g of a polyester of formula (I) as a viscous solution (yield: 71.3%).

¹H-NMR (500 MHz, CDCl₃) δ (ppm) : 2.7 (1H q), 3.2 (1H q), 4.4 (4H m), 5.6 (1H m), 6.7 (1H d).

¹³C-NMR (500 MHz, CDCl₃) δ (ppm): 37.3(lactone ring 5-position α), 62.1(alkyl), 78.3(lactone ring), 127.9(lactone ring), 155.4(lactone ring), 161.2(carboxyl group), 170.9(lactone ring), 172.7(carboxyl group).

IR (v/cm⁻¹): 2962 (alkyl chain), 1736 (lactone ring carbonyl).

20% weight reduction temperature: 290°C.

Weight-average molecular weight: ≥11,000 (tetrahydrofuran).

Glass transition temperature: -4°C.

### Example 3: Production of polyurethane

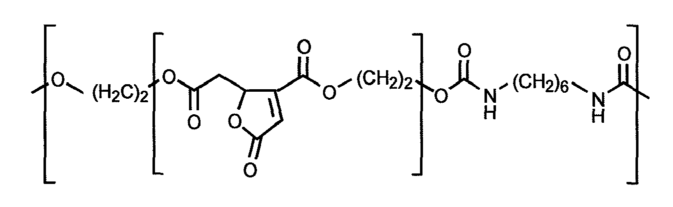

A 0.87 g portion of the polyester obtained in Example 2 was taken, 7 ml of tetrahydrofuran was added and the mixture was stirred and dissolved. After completely dissolving the polyester, 0.03 g of hexamethylene diisocyanate (an equimolar amount with respect to the polyester) was added and the mixture was stirred for 1 minute, after which several drops of tin 2-ethylhexanoate were added as a catalyst and the mixture was stirred for 24 hours. The reaction mixture was added dropwise to methanol and stirred, and the methanol was distilled off.

IR (v/cm⁻¹): 1730 (urethane carbonyl), 1750 (-lactone ring carbonyl).

Weight-average molecular weight: ≥200,000 (dimethylformamide).

### Example 4: Production of 3-carboxymuconolactone acid chloride

In a 50 ml flask there was placed 3-carboxymuconolactone (0.11 g, 0.60 mmol), and then 10 ml of chloroform was added and the mixture was stirred. After dispersing the 3-carboxymuconolactone in the chloroform, oxalyl chloride (1.0 ml, 12 mmol) was added, and then 10 µl of dimethylformamide was further added as a catalyst and the mixture was stirred for 30 minutes at room temperature under an Ar atmosphere. The 3-carboxymuconolactone gradually dissolved as the reaction proceeded, with complete dissolution after 30 minutes of stirring. Upon completion of the reaction, the excess oxalyl chloride was distilled off. Next, 5 ml of chloroform was added and distilled off (x 2) to obtain 0.13 g of a yellow viscous liquid containing an acid chloride (crude yield: 98.6%).

¹H-NMR (500 MHz, CDCl₃) δ (ppm): 3.37(1H q), 3.74(1H q), 5.55 (1H m), 7.07 (1H d).

¹³C-NMR (500 MHz, CDCl₃) δ (ppm): 47.80(lactone ring 5-position α), 76.66(lactone ring), 133.84(lactone ring), 156.82(lactone ring), 162.05(carboxyl group), 167.63(lactone ring), 169.36(carboxyl group).

### Example 5: Production of methyl 2-(2-methoxy-2-oxoethyl)-5-oxo-2,5-dihydrofuran-3-carboxylate

Following acid chloridation of 3-carboxymuconolactone (0.11 g, 0.60 mmol) in the same manner as Example 4, 5 ml of chloroform was added and the mixture was dissolved while stirring. Separately, 5 ml (an excess) of methanol was taken in a 100 ml flask, an acid chloridating solution was added thereto over a period of 30 seconds, and the mixture was stirred for 20 minutes at room temperature under an Ar atmosphere. The obtained solution was dissolved in chloroform, brine was added and the organic layer was extracted (x 3). The obtained organic layer was dried over magnesium sulfate, and the organic layer was distilled off under reduced pressure to obtain 0.11 g of the title compound as a faint yellow viscous liquid (yield: 91.6%).

¹H-NMR (500 MHz, CDCl₃) δ (ppm): 2.77 (1H q), 3.18 (1H q), 3.73(3H s), 3.93(3H s), 5.56 (1H m), 6.73 (1H d).

¹³C-NMR (500 MHz, CDCl₃) δ (ppm): 37.00(lactone ring 5-position α), 52.24(methyl group), 53.00(methyl group), 78.34(lactone ring), 127.48(lactone ring), 155.78(lactone ring), 161.19(carboxyl group), 169.12(carboxyl group), 170.13(lactone ring).

GC-MS m/z: 214 (M⁺), 182, 154, 141, 113.

### Example 6: Production of polyester

Following acid chloridation of 3-carboxymuconolactone (0.53 g, 2.82 mmol) in the same manner as Example 4, 5 ml of chloroform was added and the mixture was dissolved while stirring. Ethylene glycol (0.18 g, 2.82 mmol) was then taken separately into a 100 ml flask, 10 ml of chloroform was added and the mixture was stirred. After then adding an acid chloridating solution thereto over a period of 1 minute, the mixture was stirred for 24 hours at room temperature under an Ar atmosphere. The solvent was distilled off under reduced pressure and then dried in a vacuum at 60°C to obtain 0.52 g of a brown viscous liquid (yield: 86.3%).

¹H-NMR (500 MHz, CDCl₃) δ (ppm) : 2.7 (1H q), 3.2 (1H q), 4.4(4H m), 5.6 (1H m), 6.7 (1H d).

¹³C-NMR (500 MHz, CDCl₃) δ (ppm) : 37.3(lactone ring 5 position α), 62.1(alkyl), 78.3(lactone ring), 127.9(lactone ring), 155.4(lactone ring), 161.2(carboxyl group), 170.9(lactone ring), 172.7(carboxyl group).

IR (v/cm⁻¹): 2962 (alkyl chain), 1736 (lactone ring carbonyl).

Weight-average molecular weight: ≥30,000 (tetrahydrofuran).

## Claims

1. A polyester comprising a repeating unit represented by the following formula (I): 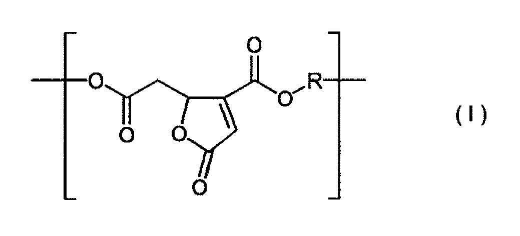 wherein R represents a hydrocarbon-based divalent residue which, in the structure, optionally contains a heteroatom without an active hydrogen.

2. A polyester according to claim 1, wherein R represents R¹, R¹-(OR¹)ₐ or R²- (O₂C-R¹-CO₂R²)_{b} wherein R¹ and R² independently of each other represent a C1-24 saturated or unsaturated hydrocarbon divalent residue, and a and b independently of each other represent an integer of 1-4.

3. A polyester according to claim 1 or 2, wherein R is a C1-24 straight- or branched-chain alkylene group.

4. A polyurethane comprising a repeating unit represented by the following formula (II): 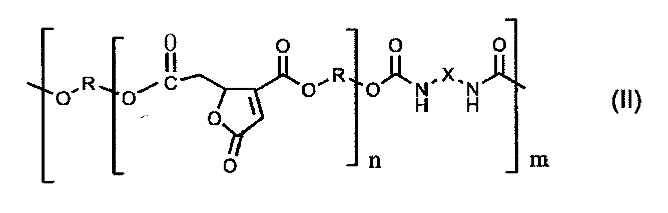 wherein
R and X independently of each other represent a hydrocarbon-based divalent residue which, in the structure, optionally contains a heteroatom without an active hydrogen; and m and n each represent an integer of 1 or greater.

5. A polyurethane according to claim 4, wherein R and X independently of each other represent R¹, R¹-(OR¹)ₐ or R²- (O₂C-R¹-CO₂R²)_{b} (where R¹ and R² independently of each other represent a C1-24 saturated or unsaturated hydrocarbon divalent residue; and a and b independently of each other represent an integer of 1-4).

6. A polyurethane according to claim 4 or 5,
wherein R and X are independently of each other a C1-24 straight- or branched-chain alkylene group.

7. A process for production of a polyester comprising a repeating unit represented by the following formula (I): 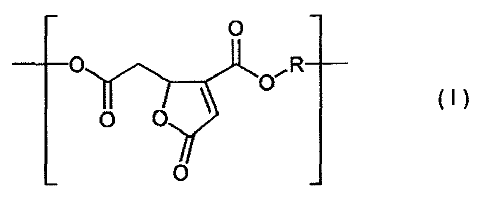 wherein R represents a hydrocarbon-based divalent residue which, in the structure, optionally contains a heteroatom without an active hydrogen,
wherein the process comprises polycondensation of 3-carboxymuconolactone or its salt or halide, with a diol or an alkali metal addition product thereof.

8. A process for production of a polyurethane comprising a repeating unit represented by the following formula (II): 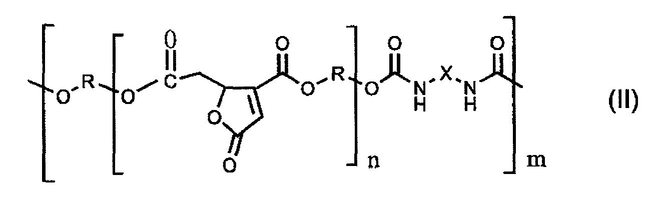 wherein R and X independently of each other represent a hydrocarbon-based divalent residue which, in the structure, optionally contains a heteroatom without an active hydrogen; and m and n each represent an integer of 1 or greater,
in which a polyester obtained by the production process according to claim 7 is reacted with a diisocyanate.

9. A compound represented by the following formula (III): 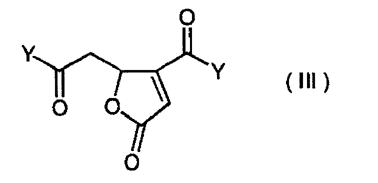 wherein Y represents a C1-6 alkoxy group, a halogen atom or a -O-R-OH group; and R represents a hydrocarbon-based divalent residue which, in the structure, optionally contains a heteroatom without an active hydrogen.
